# FASCICULE DE BREVET EUROPEEN

(11) **EP 0 885 656 B1**
(45) Date de publication et mention de la délivrance du brevet: **02.01.2002**
(21) Numéro de dépôt: 98401405.0
(22) Date de dépôt: 10.06.1998
(51) Int. Cl.: B01J 31/14

(54) **Composition catalytique et procédé pour l'oligomérisation de l'éthylène, en particulier en butène-1 et/ou hexène-1**
Katalytische Zusammensetzung und Ethylenoligomerisierung, insbesondere in 1-buten und/oder 1-hexen
Catalytic composition and ethylene oligomerization, especially in 1-butene and/or 1-hexene

(30) Priorité: 17.06.1997 FR 9707613
(43) Date de publication de la demande: 23.12.1998
(73) Titulaire: INSTITUT FRANCAIS DU PETROLE, 92500 Rueil Malmaison (FR)
(72) Inventeur: Commereuc, Dominique, 92190 Meudon (FR); Drochon, Sébastien, 92500 Rueil Malmaison (FR); Saussine, Lucien, 78290 Croissy sur Seine (FR)

(56) Documents cités:
- EP-A- 0 706 983
- EP-A- 0 769 323
- DE-A- 19 607 888
- FR-A- 1 453 329

## Description

La présente invention concerne un procédé d'oligomérisation de l'éthylène, en particulier en butène-1 et/ou hexène-1, et la composition catalytique utilisée.

Les procédés de production d'alpha oléfines à partir d'éthylène conduisent en général à un ensemble d'oligomères ayant un nombre de carbone compris entre 4 et 30 et même supérieur à 30 et les oléfines sont ensuite séparées par distillation. Depuis quelques années est apparue une demande croissante en oligomères inférieurs, essentiellement butène-1, hexène-1 et octène-1, qui sont utilisés comme comonomères avec l'éthylène dans la fabrication du polyéthylène basse densité linéaire.

Il existe peu de catalyseurs conduisant sélectivement à la formation d'un oligomère particulier comme c'est le cas dans la dimérisation de l'éthylène en butène-1 avec un catalyseur à base de titane. Il est cependant connu que des catalyseurs à base de chrome peuvent conduire à la formation d'hexène-1 principalement, avec plus ou moins de polyéthylène, la proportion des butènes et des octènes dans les produits étant très faible (R. M. Manyik, W. E. Walker, T. P. Wilson, J. Catal., 1977, 47, 197 et J. R. Briggs, J. Chem. Soc., Chem. Commun. 1989, 674 et références citées). Des catalyseurs permettant la trimérisation sélective de l'éthylène ont été revendiqués récemment (US 5 198 563, US 5 288 823, US 5 382 738, EP 608 447, EP 611 743, EP 614 865). Ces catalyseurs sont préparés à partir d'un sel de chrome et d'un amidure métallique, un pyrrolure en particulier. D'autres catalyseurs font intervenir un aluminoxane et un complexe du chrome avec une phosphine chélatante (US 5 550 305).

Il a maintenant été trouvé selon la présente invention qu'une composition catalytique obtenue en mélangeant au moins un composé de chrome avec au moins un composé aryloxy d'aluminium et avec au moins un composé d'aluminium hydrocarbyl présente une sélectivité particulière pour la formation de butène-1 et/ou d'hexène-1 par oligomérisation de l'éthylène.

Plus précisément, ladite composition catalytique améliorée est obtenue par mélange :
- d'au moins un composé de chrome pouvant comporter un ou plusieurs anions identiques ou différents par exemple choisis dans le groupe formé par les halogénures, carboxylates, acétylacétonates, les anions alcoxy, aryloxy,
- avec au moins un composé aryloxy d'aluminium de formule générale RₙAl(R'O)₃₋ₙ dans laquelle n est un nombre entier qui peut prendre les valeurs 0, 1 ou 2, R est un radical hydrocarbyle, linéaire ou ramifié, comportant de 1 à 30 atomes de carbone, et R'O est un radical aryloxy contenant de 6 à 80 atomes de carbone,
- et avec au moins un composé d'aluminium hydrocarbyl de formule générale AlR"ₘX₃₋ₘ dans laquelle R" est un radical hydrocarbyle comprenant de 1 à 6 atomes de carbone, X est un atome de chlore ou de brome et m est un nombre compris entre 1 et 3.

Le composé de chrome peut être un sel de chrome(II) ou de chrome(III) mais aussi un sel de degré d'oxydation différent pouvant comporter un ou plusieurs anions identiques ou différents tels que par exemple des halogénures, des carboxylates, des acétylacétonates, des anions alcoxy, aryloxy. Les composés de chrome utilisés de préférence dans l'invention sont les composés du chrome(III) car ils sont plus accessibles, mais un composé de chrome(I) ou de chrome(II) peut aussi convenir.

Les composés du chrome choisis peuvent être avantageusement solubilisés en milieu hydrocarboné par complexation avec un composé oxygéné organique tel que un éther ou un ester ou un composé choisi dans la classe des acétals et des cétals, résultant de la condensation d'un aldéhyde ou d'une cétone avec un monoalcool ou un polyalcool, tel que par exemple le di-(éthyl-2-hexyloxy)-2,2-propane.

Le composé aryloxy d'aluminium est choisi parmi les composés aryloxy d'aluminium de formule générale RₙAl(R'O)₃₋ₙ dans laquelle n est un nombre entier qui peut prendre les valeurs 0, 1 ou 2. R est un radical hydrocarbyle, linéaire ou ramifié, comportant de 1 à 30 atomes de carbone, par exemple alkyle, cycloalkyle, alkényle, aryle, ou aralkyle, aryle ou cycloalkyle substitué, de préférence un reste hydrocarbyle de 2 à 10 atomes de carbone. A titre d'exemple, et sans que la liste soit limitative, R peut être un reste éthyle, n-propyle, isopropyle, n-butyle, isobutyle, cyclohexyle, benzyle, diphénylméthyle, phényle, méthyl-2-phényle, diphényl-2,6-phényle. R'O est un radical aryloxy contenant de 6 à 80 atomes de carbone.

Les composés aryloxy d'aluminium préférés comportent un radical aryloxy R'O qui a pour formule générale : 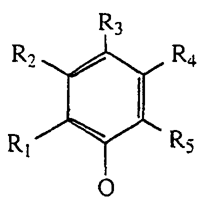 dans laquelle R₁, R₂, R₃, R₄, R₅, identiques ou différents, représentent un hydrogène, un halogène ou un radical hydrocarbyle, par exemple alkyle, cycloalkyle, alkényle, aryle, ou aralkyle, aryle ou cycloalkyle substitué, comprenant de préférence de 1 à 16 atomes de carbone, et particulièrement de 1 à 10 atomes de carbone. A titre d'exemple, et sans que la liste soit limitative, R₁, R₂, R₃, R₄, R₅ peuvent être un reste méthyle, éthyle, n-propyle, isopropyle, n-butyle, tert-butyle, cyclohexyle, benzyle, phényle, 2-méthyl-2-phénylprop-1-yle.

Parmi les radicaux aryloxy préférés, on peut citer à titre d'exemples non limitatifs: le 4-phénylphénoxy, le 2-phénylphénoxy, le 2,6-diphénylphénoxy, le 2,4,6-triphénylphénoxy, le 2,3,5,6-tétraphénylphénoxy, le 2,4-ditert-butyl-6-phénylphénoxy, le 2,6-diisopropylphénoxy, le 2,6-diméthylphénoxy,le 2,6-ditert-butylphénoxy, le 4-méthyl-2,6-ditert-butylphénoxy, le 2,6-dichloro-4-tert-butylphénoxy, le 2,6-dibromo-4-tert-butylphénoxy. Lorsque le composé aryloxy d'aluminium hydrocarbyl est choisi parmi les aluminium aryloxydes de formule générale RAl(R'O)₂ les deux radicaux aryloxy peuvent être portés par une même molécule comme par exemple le radical biphénoxy, le binaphtoxy ou le 1,8-naphtalène-dioxy, substitués ou non par des radicaux alkyle, aryle, ou halogénure.

Sont particulièrement préférés le bis(2,6-diphénylphénoxy)-aluminium isobutyle ou le bis(2,6-diphénylphénoxy)-aluminium éthyle.

La préparation du composé RₙAl(R'O)₃₋ₙ est connue dans la littérature. Tout procédé de préparation de ce composé peut convenir comme par exemple la réaction d'un phénol R'OH avec un trialkylaluminium AIR₃ dans un solvant organique, par exemple un hydrocarbure ou un éther.

Les composés d'aluminium hydrocarbyl utilisés dans l'invention sont représentés par la formule générale AlR"ₘX₃₋ₘ dans laquelle R" est un radical hydrocarbyle, de préférence alkyle comprenant de 1 à 6 atomes de carbone, X est un atome de chlore ou de brome, de préférence un atome de chlore et m est un nombre compris entre 1 et 3. On peut citer à titre d'exemples non limitatifs: le dichloroéthylaluminium, le sesquichlorure d'éthylaluminium, le chlorodiéthylaluminium, le chlorodiisobutylaluminium, le triéthylaluminium, le tripropylaluminium, le triisobutylaluminium. Le composé d'aluminium hydrocarbyl préféré est le triéthylaluminium.

Les composants du catalyseur peuvent être mis en contact dans un solvant constitué par un hydrocarbure saturé comme l'hexane, le cyclohexane, l'heptane, le butane, l'isobutane, par un hydrocarbure insaturé comme une monooléfine ou une dioléfine comportant par exemple de 4 à 20 atomes de carbone, ou par un hydrocarbure aromatique tel que le benzène, le toluène, le xylène, le mésitylène, l'éthylbenzène, purs ou en mélange.

La concentration du chrome dans la solution catalytique peut varier de 1.10⁻⁵ à 0.1 mole/L, de préférence de 5.10⁻⁵ à 1.10⁻² mole/L. Le rapport molaire entre le composé aryloxy d'aluminium et le composé de chrome peut varier entre 1:1 et 30:1, de préférence entre 1:1 et 20:1. Le rapport molaire entre l'aluminium hydrocarbyl et le composé de chrome est choisi entre 1:1 et 35:1, de préférence entre 1:1 et 15:1.

L'ordre de mélange des trois constituants de la composition catalytique n'est pas critique. Cependant, on préfère mélanger d'abord le composé du chrome avec le composé aryloxy d'aluminium, et ajouter ensuite le composé d'aluminium hydrocarbyl.

La réaction d'oligomérisation de l'éthylène peut être effectuée sous une pression totale comprise entre 0,5 et 15 MPa, de préférence entre 1 et 8 MPa, et à une température comprise entre 20 et 180°C, de préférence entre 50 et 160°C.

Dans un mode particulier de mise en oeuvre de la réaction catalytique d'oligomérisation en discontinu, on introduit un volume choisi de la solution catalytique, préparée comme décrit ci-dessus, dans un réacteur muni des dispositifs habituels d'agitation, de chauffage et de refroidissement, puis on pressurise par de l'éthylène à la pression désirée, et on ajuste la température à la valeur souhaitée. Le réacteur d'oligomérisation est maintenu à pression constante par introduction d'éthylène jusqu'à ce que le volume total de liquide produit représente, par exemple, entre 2 et 50 fois le volume de la solution catalytique primitivement introduit. On détruit alors le catalyseur par tout moyen habituel connu de l'homme de l'art, puis on soutire et on sépare les produits de la réaction et le solvant.

En cas d'opération en continu, la mise en oeuvre est de préférence la suivante : la solution catalytique est injectée en même temps que l'éthylène dans un réacteur agité par les moyens mécaniques classiques ou par une recirculation extérieure, et maintenu à la température souhaitée. On peut aussi injecter séparément les composants du catalyseur dans le milieu réactionnel, par exemple le produit d'interaction du composé du chrome avec le composé aryloxy d'aluminium de formule RₙAl(R'O)₃₋ₙ d'une part, et le composé d'aluminium hydrocarbyl représenté par la formule générale AlR"ₘX₃₋ₘ d'autre part. L'éthylène est introduit par une vanne d'admission asservie à la pression, qui maintient celle-ci constante. Le mélange réactionnel est soutiré au moyen d'une vanne asservie au niveau liquide de façon à maintenir celui-ci constant. Le catalyseur est détruit en continu par tout moyen habituel connu de l'homme de l'art, puis les produits de la réaction ainsi que le solvant sont séparés, par exemple par distillation. L'éthylène qui n'a pas été transformé peut être recyclé dans le réacteur.

Les exemples suivants illustrent l'invention sans en limiter la portée.

### EXEMPLE 1

Dans un ballon en verre de 100 mL placé sous atmosphère inerte, on introduit à l'abri de l'humidité 0,5 10⁻³ mole d'éthyl-2-hexanoate de chrome(III) en solution dans une huile minérale, cette solution est chauffée sous vide 10⁻² torr (1,33 Pa) pendant une heure puis diluée avec 25 mL de toluène distillé sous atmosphère inerte.

Dans un autoclave en acier inoxydable d'un volume utile de 100 mL, muni d'une double enveloppe permettant de réguler la température par circulation d'huile, on introduit , dans l'ordre, sous d'éthylène, et à la température ambiante, 5 mL de la solution d'éthyl-2-hexanoate de chrome(III) préparée ci-dessus, soit 0,1 10⁻³ mole de chrome, 8 mL de solution 0,1 M de bis(2,6-diphénylphénoxy)-isobutylaluminium dans le toluène, soit 0,8 10⁻³ mole, et 0,8 10⁻³ mole de triéthylaluminium en solution dans 8 mL de toluène. La température est alors portée à 120°C et la pression d'éthylène est maintenue à 5 MPa.

Après une heure de réaction, l'introduction d'éthylène est arrêtée et le catalyseur est désactivé par injection de 0,5 mL d'éthanol en solution dans 1,5 mL de toluène, au moyen d'un sas que l'on peut porter à une pression supérieure à celle de l'autoclave. Le réacteur est refroidi et dégazé puis le gaz et le liquide sont analysés par chromatographie en phase vapeur. On a consommé 19 g d'éthylène en une heure. La composition des produits est donnée dans le tableau 1. On recueille en outre 18% poids de polymère solide par rapport à l'éthylène consommé.

### EXEMPLE 2

Dans le même appareillage que celui qui a été utilisé pour l'exemple 1 et dans les mêmes conditions, à ceci près que l'on a introduit deux fois moins de bis(di-phényl-2,6-phénoxy)-isobutylaluminium, on a consommé 12 g d'éthylène en une heure de réaction. La composition des produits est donnée dans le tableau 1. On recueille en outre 30 % poids de polymère solide par rapport à l'éthylène consommé.

### EXEMPLE 3 (comparatif)

Dans le même appareillage que celui qui a été utilisé pour l'exemple 1 et dans les mêmes conditions, à ceci près que l'on a pas introduit de bis(diphényl-2,6-phénoxy)-isobutylaluminium, on a consommé 1 g d'éthylène en une heure de réaction. La composition des produits est donnée dans le tableau 1. On recueille en outre 72 % poids de polymère par rapport à l'éthylène consommé.

### EXEMPLE 4

Dans le même appareillage que celui qui a été utilisé pour l'exemple 1 et dans les mêmes conditions, à ceci près que l'on opère à 3 MPa et que le rapport molaire bis(di-phényl-2,6-phénoxy)-isobutylaluminium/Cr est de 5/1, on a consommé 14 g d'éthylène en une heure de réaction. La composition des produits est donnée dans le tableau 1. On recueille en outre 20 % poids de polymère solide par rapport à l'éthylène consommé.

### EXEMPLE 5

Dans le même appareillage que celui qui a été utilisé pour l'exemple 1 et dans les mêmes conditions, à ceci près que le bis(di-phényl-2,6-phénoxy)-isobutylaluminium a été remplacé par le bis(di-tert-butyl-2,6-phénoxy)-isobutylaluminium, on a consommé 5 g d'éthylène en une heure de réaction. La composition des produits est donnée dans le tableau 1. On recueille en outre 35 % poids de polymère solide par rapport à l'éthylène consommé.

### EXEMPLE 6

Dans le même appareillage que celui qui a été utilisé pour l'exemple 1 et dans les mêmes conditions, à ceci près que l'on opère à 3 MPa, que le rapport molaire bis(di-phényl-2,6-phénoxy)-isobutylaluminium/Cr est de 10/1 et que le rapport molaire triéthylaluminium/Cr est de 5/1, on a consommé 5 g d'éthylène en une heure de réaction. La composition des produits est donnée dans le tableau 1. On recueille en outre 40 % poids de polymère solide par rapport à l'éthylène consommé.

### EXEMPLE 7

Dans le même appareillage que celui qui a été utilisé pour l'exemple 1 et dans les mêmes conditions, à ceci près que l'on remplace le bis(di-phényl-2,6-phénoxy)-isobutylaluminium par le di-phényl-2,6-phénoxy-diéthyl-aluminium, que le rapport molaire di-phényl-2,6-phénoxy-diéthylaluminium/Cr est de 4/1 et que le rapport molaire triéthylaluminium/Cr est de 10/1, on a consommé 5,4 g d'éthylène en une heure de réaction. La composition des produits est donnée dans le tableau 1. On recueille en outre 41 % poids de polymère solide par rapport à l'éthylène consommé.

**TABLEAU I**

| | Répartition des produits obtenus (% poids) | | | | Teneur en oléfines alpha (% poids) | | |
|---|---|---|---|---|---|---|---|
| Exemple | C₄ | C₆ | C₈ | C₁₀+ | dans C₄ | dans C₆ | dans C₈ |
| 1 | 0,4 | 74,5 | 0,9 | 6,2 | 87,2 | 99,8 | 96,5 |
| 2 | 5,5 | 60,6 | 1,2 | 2,7 | 92,7 | 99,6 | 95,7 |
| 3 | 19,1 | 6,5 | 0,4 | 2 | 79,3 | 55,3 | |
| 4 | 0,4 | 67,5 | 0,7 | 11,4 | 98,1 | 99,7 | |
| 5 | 18.5 | 42.6 | 1 | 2.9 | 94.4 | 95.9 | 75.3 |
| 6 | 22 | 38 | 0 | 0 | 99.4 | 99.2 | |
| 7 | 49.5 | 8.5 | 0.6 | 0.7 | 98.0 | 96.9 | |

En fonction de la composition catalytique qui est choisie, le procédé selon l'invention produit donc essentiellement du butène-1 et/ou de l'hexène-1 ou leurs mélanges, à l'exclusion des oléfines supérieures, et avec une sélectivité alpha élevée

## Revendications

1. Composition catalytique obtenue par mélange :
- d'au moins un composé de chrome,
- avec au moins un composé aryloxy d'aluminium de formule générale RₙAl(R'O)₃₋ₙ dans laquelle n est un nombre entier qui peut prendre les valeurs 0, 1, ou 2, R est un radical hydrocarbyle, linéaire ou ramifié, comportant de 1 à 30 atomes de carbone, et R'O est un radical aryloxy contenant de 6 à 80 atomes de carbone,
- et avec au moins un composé d'aluminium hydrocarbyl de formule générale AlR"ₘX₃₋ₘ dans laquelle R" est un radical hydrocarbyle comprenant de 1 à 6 atomes de carbone, X est un atome de chlore ou de brome et m est un nombre compris entre 1 et 3.

2. Composition selon la revendication 1, **caractérisée en ce que** le composé de chrome comporte un ou plusieurs anions identiques ou différents choisis dans le groupe formé par les halogénures, carboxylates, acétylacétonates, les anions alcoxy, aryloxy.

3. Composition selon l'une des revendications 1 ou 2, **caractérisée en ce que** dans le composé aryloxy d'aluminium de formule générale RₙAl(R'O)₃₋ₙ, le radical aryloxy R'O a pour formule générale 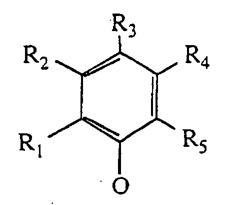 dans laquelle R₁, R₂, R₃, R₄, R₅, identiques ou différents, représentent un hydrogène, un halogène ou un radical hydrocarbyle comprenant de 1 à 16 atomes de carbone.

4. Composition selon l'une des revendications 1 à 3, **caractérisée en ce que** le composé aryloxy d'aluminium est le bis(2,6-diphénylphénoxy)-aluminium isobutyle ou le bis(2,6-diphénylphénoxy)-aluminium éthyle.

5. Composition selon l'une des revendications 1 à 4, **caractérisée en ce que** le composé d'aluminium hydrocarbyl est le dichloroéthylaluminium, le sesquichlorure d'éthylaluminium, le chlorodiéthylaluminium, le chlorodiisobutylaluminium, le triéthylaluminium, le tripropylaluminium, le triisobutylaluminium.

6. Composition selon l'une des revendications 1 à 4, **caractérisée en ce que** le composé d'aluminium hydrocarbyl est le triéthylaluminium.

7. Composition selon l'une des revendications 1 à 6, **caractérisée en ce que** les composants du catalyseur sont mis en contact dans un solvant constitué par un hydrocarbure saturé, insaturé oléfinique ou dioléfinique, ou aromatique.

8. Composition selon l'une des revendications 1 à 7, **caractérisée en ce que** la concentration du chrome dans la solution catalytique est comprise entre 1.10⁻⁵ et 0.1 mole/L.

9. Composition selon l'une des revendications 1 à 8, **caractérisée en ce que** le rapport molaire entre le composé aryloxy d'aluminium et le composé de chrome est compris entre 1:1 et 30:1 et le rapport molaire entre l'aluminium hydrocarbyl et le composé de chrome est compris entre 1:1 et 35:1.

10. Procédé d'oligomérisation de l'éthylène avec une composition catalytique selon l'une des revendications 1 à 9.

11. Procédé selon la revendications 10, **caractérisé en ce que** la réaction d'oligomérisation de l'éthylène est effectuée sous une pression comprise entre 0,5 et 15 MPa et à une température comprise entre 20 et 180°C.

## Patentansprüche

1. Katalytische Zusammensetzung, die erhalten wird durch Mischen:
- von wenigstens einer Chromverbindung
- mit wenigstens einer Aryloxyverbindung von Aluminium der allgemeinen Formel RₙAl(R'O)₃₋ₙ, in der n eine ganze Zahl ist, die Werte 0, 1 oder 2 annehmen kann, R ein lineares oder verzweigtes Hydrocarbylradikal ist, das 1 bis 30 Kohlenstoffatome aufweist, und R'O ein Aryloxyradikal ist, das 6 bis 80 Kohlenstoffatome enthält,
- und mit wenigstens einer Hydrocarbylverbindung von Aluminium der allgemeinen Formel AlR"ₘX₃₋ₘ, in der R" ein Hydrocarbylradikal ist, das 1 bis 6 Kohlenstoffatome umfasst, X ein Chlor- oder Bromatom ist und m eine Zahl zwischen 1 und 3 ist.

2. Zusammensetzung nach Anspruch 1, **dadurch gekennzeichnet, dass** die Chromverbindung ein oder mehrere identische oder verschiedene Anionen umfasst, die aus der Gruppe gewählt sind, die gebildet wird durch die Halogenide, die Carboxylate, die Acetylacetonate, die Alkoxy-, Aryloxyanionen.

3. Zusammensetzung nach einem der Ansprüche 1 oder 2, **dadurch gekennzeichnet, dass** in der Aryloxyverbindung von Aluminium der allgemeinen Formel RₙAl(R'O)₃₋ₙ, das Aryloxyradikal R'O, als allgemeine Formel hat: 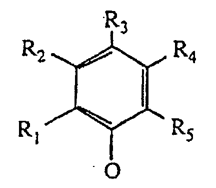 in welcher R₁, R₂, R₃, R₄, R₅, identisch oder verschieden ein Wasserstoff-, ein Halogen- oder ein Hydrocarbylradikal darstellen, das 1 bis 16 Kohlenstoffatome umfasst.

4. Zusammensetzung nach einem der Ansprüche 1 bis 3, **dadurch gekennzeichnet, dass** die Aryloxyverbindung von Aluminium bis(2,6-Dipheylphenoxy)-Isobutylaluminium oder bis(2,6-Diphenylphenoxy)-Ethylaluminium ist.

5. Zusammensetzung nach einem der Ansprüche 1 bis 4, **dadurch gekennzeichnet, dass** die Hydrocarbylverbindung von Ddicholorethylaluminium, Ethylaluminiumsesquichlorid, Chlordiethylaluminium, Chlordiisobutylaluminium, Triethylaluminium, Tripropylaluminium, Triisobutylaluminium ist.

6. Zusammensetzung nach einem der Ansprüche 1 bis 4, **dadurch gekennzeichnet, dass** die Hydrocarbylverbindung von Aluminium Triethylaluminium ist.

7. Zusammensetzung nach einem der Ansprüche 1 bis 6, **dadurch gekennzeichnet, dass** die Bestandteile des Katalysators in einem Lösungsmittel in Kontakt gebracht werden, das aus einem gesättigten, ungesättigten olefinischen, diolefinischen oder aromatischen Kohlenwasserstoff besteht.

8. Zusammensetzung nach einem der Ansprüche 1 bis 7, **dadurch gekennzeichnet, dass** die Chromkonzentration in der katalytischen Lösung zwischen 1.10⁻⁵ und 0,5 mol/l liegt.

9. Zusammensetzung nach einem der Ansprüche 1 bis 8, **dadurch gekennzeichnet, dass** das molare Verhältnis zwischen der Aryloxyverbindung von Aluminium und der Chromverbindung zwischen 1:1 und 30:1 liegt und das molare Verhältnis zwischen Aluminiumhydrocarbyl und der Chromverbindung zwischen 1:1 und 35:1 liegt.

10. Verfahren zur Oligomerisierung von Ethylen mit einer katalytischen Zusammensetzung gemäß einem der Ansprüche 1 bis 9.

11. Verfahren nach Anspruch 10, **dadurch gekennzeichnet, dass** die Oligomerisierungsreaktion von Ethylen unter einem Druck durchgeführt wird, der zwischen 0,5 und 15 MPa liegt und bei einer Temperatur, die zwischen 20 und 180°C liegt.

## Claims

1. A catalytic composition obtained by mixing:
• at least one chromium compound;
• with at least one aryloxy aluminium compound with general formula RₙAl(R'O)₃₋ₙ in which n is a whole number which can take the values 0, 1 or 2, R is a linear or branched hydrocarbyl radical containing 1 to 30 carbon atoms, and R'O is an aryloxy radical containing 6 to 80 carbon atoms;
• and with at least one hydrocarbyl aluminium compound with general formula AlR"ₘX₃₋ₘ where R" is a hydrocarbyl radical containing 1 to 6 carbon atoms, X is a chlorine or bromine atom and m is a number from 1 to 3.

2. A composition according to claim 1, **characterized in that** the chromium compound comprises one or more identical or different anions selected from the group formed by halides, carboxylates, acetylacetonates, and alkoxy and aryloxy anions.

3. A composition according to claim 1 or claim 2, **characterized in that** in the aryloxy aluminium compound with general formula RₙAl(R'O)₃₋ₙ, the aryloxy radical R'O has general formula: 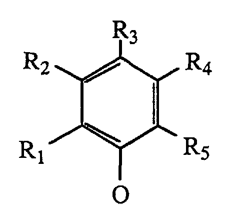 where R₁, R₂, R₃, R₄, R₅, which may be identical or different, represent a hydrogen, a halogen or a hydrocarbyl radical containing 1 to 16 carbon atoms.

4. A composition according to any one of claims 1 to 3, **characterized in that** the aryloxy aluminium compound is bis(2,6-diphenylphenoxy) isobutylaluminium or bis(2,6-diphenylphenoxy) ethylaluminium.

5. A composition according to any one of claims 1 to 4, **characterized in that** the hydrocarbyl aluminium compound is dichloroethylaluminium, ethylaluminium sesquichloride, chlorodiethylaluminium, chlorodiisobutylaluminium, triethylaluminium, tripropylaluminium, or triisobutylaluminium.

6. A composition according to any one of claims 1 to 4, **characterized in that** the hydrocarbyl aluminium compound is triethylaluminium.

7. A composition according to any one of claims 1 to 6, **characterized in that** the catalyst components are brought into contact in a solvent constituted by a saturated, unsaturated, olefinic or diolefinic or aromatic hydrocarbon.

8. A composition according to any one of claims 1 to 7, **characterized in that** the concentration of chromium in the catalytic solution is in the range 1 x 10⁻⁵ to 0.1 mole/l.

9. A composition according to any one of claims 1 to 8, **characterized in that** the molar ratio between the aryloxy aluminium compound and the chromium compound is in the range 1:1 to 30:1 and the mole ratio between the hydrocarbyl aluminium and the chromium compound is in the range 1:1 to 35:1.

10. An ethylene oligomerisation process using a catalytic composition according to any one of claims 1 to 9.

11. A process according to claim 10, **characterized in that** the ethylene oligomerisation reaction is carried out at a pressure in the range 0.5 MPa to 15 MPa and at a temperature in the range 20°C to 180°C.
